# EUROPEAN PATENT SPECIFICATION

(11) **EP 4 210 669 B1**
(45) Date of publication and mention of the grant of the patent: **20.08.2025**
(21) Application number: 21773094.4
(22) Date of filing: 08.09.2021
(51) Int. Cl.: A61K 9/00, A61K 9/06, A61K 47/34, A61K 47/36, A61K 47/38, A61K 47/42, A61L 26/00, A61K 36/00, A61K 36/752

(54) **PHARMACEUTICAL COMPOSITION IN THE FORM OF A HYDROGEL COMPRISING ORANGE-DERIVED EXTRACELLULAR VESICLES**
PHARMAZEUTISCHE ZUSAMMENSETZUNG IN FORM EINES HYDROGELS MIT EXTRAZELLULÄREN VESIKELN AUS ORANGEN
COMPOSITION PHARMACEUTIQUE SOUS FORME D'HYDROGEL COMPRENANT DES VÉSICULES EXTRACELLULAIRES DÉRIVÉES DE L'ORANGE

(30) Priority: 10.09.2020 IT 202000021463
(43) Date of publication of application: 19.07.2023
(73) Proprietor: Evobiotech S.r.l. in liquidazione, 10122 Torino (IT)
(72) Inventor: CAMUSSI, Giovanni, 10132 TORINO (IT); GAI, Chiara, 12045 FOSSANO (Cuneo) (IT); POMATTO, Margherita Alba Carlotta, 10045 PIOSSASCO (Torino) (IT)
(74) Representative: Rigamonti, Dorotea
(86) International application number: PCT/EP2021/074660
(87) International publication number: WO 2022/053485

(56) References cited:
- EP-A1- 3 354 257
- WO-A1-2016/166716
- US-A1- 2018 179 543
- US-A1- 2020 230 196

## Description

### Technical field

The present invention relates to a pharmaceutical composition in the form of a hydrogel comprising orange-derived extracellular vesicle (EVs) and its use in regenerative therapies.

### Background

Extracellular vesicles (EVs) are a mixed population of small particles released by all kinds of living cells. Extracellular vesicles (EVs) include microvesicles, released through the budding of the plasma membrane, and exosomes, derived from the endosomal compartment. Extracellular vesicles are referred to as "particles", "microparticles", "nanovesicles", "microvesicles" and "exosomes" (Yáñez-Mó M, et al. Biological properties of extracellular vesicles and their physiological functions. J Extracell Vesicles. 2015 May 14; 4:27066 doi: 10.3402/jev.v4.27066; Lötvall J, et al. Minimal experimental requirements for definition of extracellular vesicles and their functions: a position statement from the International Society for Extracellular Vesicles. J Extracell Vesicles. 2014 Dec 22;3:26913. doi: 10.3402/jev.v3.26913.; Harrison P, et al. Extracellular Vesicles in Health and Disease. CRC Press, pages 1-5, 2014).

EVs are formed by a phospholipid bilayer containing cytoplasmic proteins, surface receptors, lipid-interacting proteins, DNA and RNA molecules. The cargo is complex and varies based on the cell of origin. EVs can transfer their cargo to recipient cells, in this way they mediate intercellular communication activating signaling cascades and cellular responses.

Extracellular vesicles have been isolated from nearly all mammalian cell types and body fluids, and also from lower organisms, including bacteria and plants. In recent years, numerous therapeutic approaches have focused on the use of extracellular vesicles, including skin regeneration applications.

CN 2016/10983610A discloses the use of extracellular vesicles derived from mesenchymal stem cells in combination to cytokines in a spray bottle for treating epidermal damage. CN109865033A discloses the use of an external application ointment, comprising a mesenchymal stem cell exosomes and an ointment composition, for repairing a diabetic foot wound.

Recent findings have shown that also EVs derived from edible plants exhibit beneficial therapeutic effects on skin.

WO2017/052267 discloses the use of topically administered edible native plant-derived EVs to promote skin improvement in terms of wrinkle formation, moisturization, whitening, epithelial cell proliferation and collagen deposition.

WO 2019/027387 A2 discloses the use of extracellular vesicles derived from wheatpass, garlic and ginger to promote wound healing since they can induce migration of keratinocytes.

PCT/EP2020/056632 discloses plant-derived EVs, including orange-derived EVs, and their therapeutic use for the treatment of ulcers, dermatites, corneal damages, eye diseases, mucosal lesions and infective lesions.

Among the current treatments available for wounds and lesions, one of the most common medication is represented by hydrogels. Hydrogels, originally developed in the 1950s, comprise a wide variety of hydrated polymer dressings that regulate fluid exchange from the wound surface. They are used as treatment for skin wounds and lesions because they regulate the level of fluids in the wound bed and maintain a moist environment, favoring tissue repair. Notwithstanding the above-mentioned properties, hydrogels are poorly effective as therapeutic agents because they are not able to directly promote healing processes such as cellular proliferation, cellular migration or angiogenesis (Tavakoli S, Klar AS. Advanced Hydrogels as Wound Dressings. Biomolecules. 2020;10(8):E1169. Published 2020 Aug 11. doi:10.3390/biom10081169. Broussard KC, Powers JG. Wound dressings: selecting the most appropriate type. Am J Clin Dermatol. 2013;14(6):449-459. doi:10.1007/s40257-013-0046-4).

In order to overcome the limitations and drawbacks of the prior art, the present invention provides a pharmaceutical composition in the form of a hydrogel comprising orange-derived extracellular vesicles (EVs) as well as a method for the preparation of said pharmaceutical composition as defined in the appended independent claims. The dependent claims identify further advantageous features of the claimed composition and method. The subject-matter of the appended claims forms an integral part of the present description.

### Detailed description of the invention

The present inventors have found that extracellular vesicles derived from orange plants or orange fruits promote beneficial processes for skin closure, particularly angiogenesis. Moreover, experimental studies carried out by the present inventors have surprisingly revealed that orange-derived EVs combined with a hydrogel are significantly more effective in accelerating regeneration of skin lesions characterized by ischemia or impaired angiogenesis as compared to EVs or hydrogel alone.

To the inventors' knowledge, the prior art does not disclose extracellular vesicles derived from orange plants or orange fruits having a proangiogenic effect nor the therapeutic effect of complexes of these EVs with hydrogel when administered topically on wound and skin lesions characterized by ischemia or impaired angiogenesis.

Therefore, an aspect of the present invention is a pharmaceutical composition in the form of a hydrogel comprising:
(i) orange-derived extracellular vesicles (EVs);
(ii) a polymer gelling agent;
(iii) water in an amount of at least 10% by weight of the total weight of the composition; and
(iv) optionally, pharmaceutically acceptable vehicles, excipients, and/or diluents,

wherein the orange-derived EVs are derived from one or more plants of the species *Citrus sinensis* and/or one or more fruits of said one or more *Citrus sinensis* plants,
wherein the orange-derived extracellular vesicles (EVs) are enclosed by a lipid bilayer membrane and are characterized in that they have a diameter ranging from 10 to 500 nm as measured by light scattering-based Nanoparticle Tracking Analysis (NTA) and in that they show pro-angiogenic activity,
wherein the polymer gelling agent and the water form a hydrogel matrix in which the orange-derived EVs are dispersed, said EVs being releasable from the hydrogel matrix,
for use in a tissue repair and/or regenerative therapeutic method.

As used herein, the term "orange-derived extracellular vesicles" refers to nanoparticles derived from orange fruit intended as *Citrus sinensis* fruit as well as to nanoparticles derived from cells from one or more plants of the species *Citrus sinensis.* It is understood that the term *Citrus sinensis* encompasses any *Citrus sinensis* varieties, as are known in the art. Non-limiting examples of such varieties are Valencia, Navel, Red Orange, Blood Orange, Tarocco, Sweet Oranges, Moro, Sanguinello, and Newhall.

Orange-derived EVs are delimited or encapsulated by a phospholipid bilayer and carry lipids, proteins, nucleic acids and other molecules derived from the cell they are derived from. Conventionally, the extracellular vesicles have a diameter in a range of 10-1000 nm.

The present invention makes use of orange-derived extracellular vesicles (EVs) which have a diameter in the range of from 10 to 500 nm, preferably in the range of from 20 to 400 nm, even more preferably in the range of from 25 to 350 nm.

The person skilled in the art is well aware of the standard methods available for determining EV diameter.

Among such methods, Nanoparticle Tracking Analysis (NTA) is typically used to visualize and measure nanoparticles in suspension in the range from 10 - 1000 nm based on the analysis of Brownian motion. This method allows the characterization of extracellular vesicles in solution with dynamic light scattering (DLS). Each vesicle is individually but simultaneously analyzed by direct observation of diffusion. The analysis allows to determine the particle concentration and size distribution. The size is related to the Brownian motion allowing the detection of the translational diffusion diameter of a particle, the so-called hydrodynamic diameter. The rate of particle movement is linked to the viscosity of the liquid, and the temperature and size of the particle and is not influenced by particle density or refractive index. The rate of movement of particle is related to a sphere-equivalent hydrodynamic radius as calculated through the Stokes-Einstein equation. In fact, the Brownian motion of particles or molecules in suspension causes laser light to be scattered at different intensities. Analysis of these intensity fluctuations yields the velocity of the Brownian motion and hence the particle size using the Stokes-Einstein relationship.

As it is well known in the art, Transmission Electron Microscopy (TEM) is also considered a standard tool for characterizing the morphology of EVs. TEM is a microscopy method with high resolution that allows the direct visualization and characterization of EVs. Being one of the most used techniques able to visualize EVs, TEM allows the study the EV structure, morphology, membrane integrity and size.

In one preferred embodiment of the invention, the amount of orange-derived EVs in the pharmaceutical composition is in the range of from 10¹⁰ to 10¹² EVs/ml on the total volume of the composition, more preferably the amount of orange-derived EVs is 10¹¹ EVs/ml.

In another preferred embodiment of the invention, the amount of orange-derived EVs proteins in the pharmaceutical composition is in the range of from 5 µg to 500 µg/ml on the total volume of the composition, more preferably in the range of from 40 µg to 60 µg/ml on the total volume of the composition.

In one embodiment of the invention, the protein content of the EVs population is in the range of from 1 to 10³ ng/10⁹ EVs.

In another embodiment, the RNA content of the EVs is in the range of from 10 to 60 ng/10¹⁰ EVs.

The expressions "protein content" and "RNA content" encompasses both the internal and the membrane content of the EVs used in the present invention.

Examples of the lipids in the EVs used in the present invention comprise, but are not limited to, 24-Propylidene cholesterol, beta sitosterol, campesterol, dipalmitin, eicosanol and/or glycidol stearate.

The scope of the invention includes pharmaceutical compositions containing EVs derived from the fruits and/or plants of a single variety of *Citrus sinensis* as well as pharmaceutical compositions containing EVs derived from the fruits and/or plants of a plurality of *Citrus sinensis* varieties.

The EVs used in the present invention are characterized in that they show pro-angiogenic activity.

Within the present description, the expression "pro-angiogenic effect" is intended as the promotion of endothelial cells migration, proliferation or vessel formation by endothelial cells and increased release of pro-angiogenic mediators *in vitro* or *in vivo.* Angiogenesis is a fundamental biologic process and its impairment is involved in the pathogenesis of several kind of lesions, including ischemic ulcers, such as pressure ulcers, arterial ulcers, venous ulcers, diabetic ulcers, ischemic ulcers, exudative ulcers, dysmetabolic ulcers, traumatic ulcers, and mucosal lesions such as diabetic lesions. Moreover, angiogenesis is essential to provide nutrients to cells and allows tissue regeneration. Angiogenesis accelerates the regeneration of different lesions, including burns, fistulae, psoriasis, keratosis, keratitis, fissures, traumatic ulcers, mucosal lesions (such as traumatic lesions due to prothesis and mouth, decubital, genital mucosal lesions), dermatitis (including acne, eczema, seborrheic dermatitis, atopic dermatitis, contact dermatitis, dyshidrotic eczema, neurodermatitis, dermatitis herpetiformis), cellular damage induced by pro-apoptotic drugs aimed to treat pre-cancerous lesions (e.g. actinic keratosis). Accordingly, the orange-derived EVs used in the present invention are particularly useful in the treatment of the above-mentioned diseases.

Preferably, the orange-derived EVs according to the invention are purified from orange juice or culture medium of orange plant cells. Orange plant cells may be derived from leaf, fruit pulp, shoot or sprout.

Suitable purification techniques include, but are not limited to, ultracentrifugation and tangential flow filtration. The selection of the most suitable method to be used for the purification of orange-derived EVs falls within the knowledge and skills of the ordinary person of skill in the art.

In one embodiment of the invention, the orange-derived EVs in the pharmaceutical composition are freshly prepared. In another embodiment, the orange-derived EVs in the pharmaceutical composition are stored at 4°C, -20°C or -80°C prior to combination with hydrogel. In a still another embodiment, the orange-derived EVs are lyophilized prior to mix with hydrogel.

According to the present invention, the pharmaceutical composition is in the form of a hydrogel. Hydrogel is a water-swollen, and cross-linked polymeric network produced by the simple reaction of one or more monomers of a polymer gelling agent. Typically, a hydrogel matrix is composed of water and one type of polymer or a combination of different polymers. The amount of water in the pharmaceutical composition according to the invention is of at least 10% by weight of the total weight of the composition.

Illustrative non-limiting examples of polymer gelling agents suitable for use in the pharmaceutical composition of the invention include, but are not limited to, cellulose and cellulose-based polymers such as carboxymethylcellulose, sodium carboxymethylcellulose, ethylcellulose, hydroxyethylcellulose, cellulose gum, collagen, hydrolyzed collagen, chitosan, gelatin, hyaluronic acid (HA), glycerol, polyethylene glycol (PEG), polysulfated glycosaminoglycan (PSGAG), glycerine, propylene glycol and calcium alginate.

Within the scope of the present invention are hydrogel composed of water and either a single polymer or a combination of different polymers. It is understood that the polymer molecules can be used in their native form or with chemical modifications. Such components may be used individually or in combination.

Hydrogels have the advantage of being available in several different physical states including, for example, sheets, amorphous gels (dry or pre-mixed), and impregnated gauzes. Their high-water content is appealing for several biomedical applications because it mimics the highly hydrated physiological extracellular matrix environment in soft tissues. In fact, hydrogels are particularly useful for the treatment of dry wounds as they have the ability to rehydrate and maintain a moist environment. Moreover, these systems keep the wound closed, promote the removal of necrotic tissue via autolysis, and promote wound debridement. They also have a cooling effect on the wound and can decrease perceived pain. In the pharmaceutical composition according to the invention, the orange-derived EVs are dispersed and encapsulated in the hydrogel matrix and releasable from said matrix to the injured tissue in a controlled manner, i.e. gradually and over a prolonged period of time.

Moreover, hydrogel-encapsulation of the EVs preparation allows to stabilize these vesicles and protect them from degradation, thereby enhancing the therapeutic effect of the vesicles. Additionally, the pharmaceutical composition of the invention may contain suitable excipients, preservatives, solvents or diluents according to conventional method.

Illustrative excipients include, but are not limited to, panthenol, glycols, including propylene glycol, polyethylene glycol, gums, including guar gum, sugars, including lactose, dextrose, sucrose, sorbitol, mannitol, xylitol, boric acid and borates, including sodium tetraborate, glycerol, glycerin, allantoin, protamine, sorbitol carbomer or sodium carbomer, triethanolamine, urea, including imidazolidinyl urea, alginate, EDTA, sodium benzoate, calcium phosphate and calcium silicate, citric acid, acetic acid, ascorbic acid, magnesium stearate, tween, fatty acids, fatty acid alcohols, fatty acid esters, plant extracts, including Aloe Barbadensis Leaf Juice.

Exemplary preservatives include, but are not limited to, parabens, including ethyl paraben, methyl paraben, propyl paraben, formaldehyde donors including DMDM hydantoin, imidazolidinyl urea, and glutaraldehyde, phenol derivatives, benzoic acid, benzyl alcohol. Suitable solvents or diluents include, but are not limited to, purified water, ethanol and benzyl alcohol.

The pharmaceutical composition of the invention may also include surfactants, humectants, antioxidants, viscosity stabilizers, chelating agents, buffers, lower alcohols, fragrances, pH regulators and the like. These molecules can be used in their native form or with chemical modifications. Such components may be used individually or in combination.

According to the invention, the pharmaceutical composition may further comprise one or more antimicrobial agents selected from antibiotics, antibacterial and antifungal agents, in order to improve the efficacy of the pharmaceutical composition in promoting the closure of the lesion. Suitable antibiotics, antifungal and antibacterial agents include, but are not limited to, ionized silver (Ag+), including silver oxide, silver nitrate, silver sulfate, silver salt, silver zeolite, silver sulfadiazine (SSD), and silver nanoparticles (AgNPs), povidone iodine, cadexamer iodine, chlorhexidine gluconate, quinolone and fluoroquinolone, including ciprofloxacin, ofloxacin, levofloxacin, penicillin, including amoxicillin, dicloxacillin, cephalosporine, including Cephalexin, lincosamide, including Clindamycin, tetracycline, including doxycycline, EDTA, trimethoprim-sulfamethoxazole, streptomycin, clindamycin and nitrofurazone and gentamicin. These molecules can be used in their native form or with chemical modifications. Such components may be used individually or in combination.

To improve the efficacy in promoting the closure of the wound, the pharmaceutical composition of the invention may additionally comprise anti-inflammatory agents. Suitable anti-inflammatory agents include, but are not limited to, steroid anti-inflammatory agents such as cortisone, hydrocortisone and prednisone, and nonsteroidal anti-inflammatory agents, including ibuprofen, naproxen, diclofenac, salicylic acid, indomethacin. Such components may be used individually or in combination.

Additionally, the pharmaceutical composition may contain growth factors and pro-regenerative agents in order to improve its therapeutic effect. Suitable growth factors and pro-regenerative agents include, but are not limited to, epidermal growth factor (EGF), vascular endothelial growth factor (VEGF), platelet-derived growth factor (PDGF), and fibroblast growth factor (FGF).

Preferably, the pharmaceutical composition according to the invention has an acidic pH comprised between 3.5 and 7.0, more preferably between 4.0 and 5.0, in order to lower the pH of the wound, prevent alkalosis, prevent infections, and promote wound closure.

The pharmaceutical composition of the invention is administrable in several ways depending on the target site. For cutaneous and external mucosal repair, the pharmaceutical composition can be administered topically or by local injection. For topical administration, the pharmaceutical composition may be formulated in a gel, ointment, cream, or salve. Preferably, the pharmaceutical composition has a soft viscous texture to improve adhesion to the wound without leaking.

In order to facilitate EV-hydrogel delivery and control the release of EVs over time, the pharmaceutical composition can be combined with medical devices, such as patches, surgical threads, gauze.

The administration dose and the rate and duration of EVs release are determined according to various factors, such as the condition to be treated, the patient's characteristics and the size of the lesion(s), and can be determined by a person of ordinary skill in the art by using his/her normal knowledge.

Preferably, the release of the orange-derived EVs from the hydrogel matrix is performed over a period of at least one day (24 hours), for example over a period of at least two days (48 hours), at least three days (72 hours), at least four days, at least five days, at least six days, at least seven days or at least eight days.

Where the pharmaceutical composition is administered topically, it should preferably be distributed to cover entirely the surface of the wound and, optionally, it should be covered with a secondary dressing to maintain the wound clean.

The pharmaceutical composition may be packaged as single unit dose, or as a plurality of single unit doses, preferably in a plastic or aluminum tube. Such tube can be provided with a small spout that allows the distribution of the gel into the bed of the wound. The pharmaceutical composition is intended to enhance cellular and/or tissue repair.

As it will be illustrated in detail in the following examples, the pharmaceutical composition of the present invention combines the therapeutic effects of orange-derived pro-angiogenic EVs and a hydrogel, surprisingly achieving an improved synergistic regenerative effect on damaged tissues. Moreover, the pharmaceutical composition of the invention guarantees the stabilization and storage of EVs as well as a local efficient and time-controlled release of EVs to the site of the lesion.

These features make the pharmaceutical composition of the invention particularly suitable for the therapeutic treatment of several kinds of lesions in damaged tissues such as skin and mucous membranes, particularly ulcers, dermatites, corneal damages, eye diseases, mucosal lesions and infective lesions. The invention provides the applicability of orange-derived EVs combined in a hydrogel as a therapeutic topic treatment promoting a regenerative effect on damaged tissues and cellular repair, particularly when the damaged tissue shows impaired angiogenesis or angiogenesis promotion accelerates the regeneration in damaged tissues. In one embodiment, the pharmaceutical composition of the invention is used in a regenerative therapeutic method for the closure of a skin, mucous membranes or soft-tissue lesion, for example for the closure of a skin wound.

Diseases and conditions wherein damaged tissues show impaired angiogenesis include, but are not limited to, ischemic ulcers, such as pressure ulcers, arterial ulcers, venous ulcers, diabetic ulcers, ischemic ulcers, exudative ulcers, dysmetabolic ulcers, traumatic ulcers, and mucosal lesions such as diabetic lesions.

Diseases and conditions wherein the promotion of angiogenesis in damaged tissues accelerates tissue regeneration include, but are not limited to, burns, fistulae, psoriasis, keratosis, keratitis, fissures, traumatic ulcers, mucosal lesions (such as traumatic lesions due to prothesis and such, mouth, decubital, genital mucosal lesions), dermatitis (including acne, eczema, seborrheic dermatitis, atopic dermatitis, contact dermatitis, dyshidrotic eczema, neurodermatitis, dermatitis herpetiformis), cellular damage induced by pro-apoptotic drugs aimed to treat pre-cancerous lesions (e.g. actinic keratosis).

Within the scope of the present invention is also a pharmaceutical composition for use as above defined, prepared by a method comprising the steps of:
(i) dissolving a polymer gelling agent in a predetermined amount of water to form a hydrogel matrix, wherein the pre-determined amount of water represents at least 10% by weight of the total weight of the pharmaceutical composition;
(ii) adding orange-derived extracellular vesicles (EVs) as above defined to the hydrogel matrix obtained in step (i); and
(iii) mixing said orange-derived EVs with the hydrogel matrix, thereby obtaining the pharmaceutical composition in the form of a hydrogel in which the EVs are dispersed in the hydrogel matrix.

Suitable polymer gelling agents for use in the method according to the invention are as above described with reference to the pharmaceutical composition.

Preferably, the polymer gelling agent is dissolved in the pre-determined amount of water at a concentration comprised within the range of from 1 to 100 mg/ml on the total volume of water, more preferably from 2 to 50 mg/ml on the total volume of water.

According to the method of the invention, the mixing of the orange-derived EVs with the hydrogel matrix in step (ii) may be performed by vortexing, preferably for a period of time of at least 30 seconds.

The method of the invention may further comprise a step (iv) of keeping the composition containing the orange-derived EVs dispersed in the hydrogel matrix at 37°C for a period of time ranging from 5 to 10 minutes, after mixing.

In one embodiment of the method of the invention, in step (i) the polymer gelling agent is mechanically stirred in water at 37°C for at least 30 minutes to obtain total dissolution of said polymer gelling agent.

In another embodiment, the hydrogel and the orange-derived EVs are kept at 37°C for at least 10 minutes before mixing.

In still another embodiment, after the mixing of step (iii), the obtained composition is kept at 37°C for at least 10 minutes.

### EXAMPLES

The following experimental section is provided purely by way of illustration and is not intended to limit the scope of the invention as defined in the appended claims. In the following experimental section, reference is made to the appended drawings, wherein:

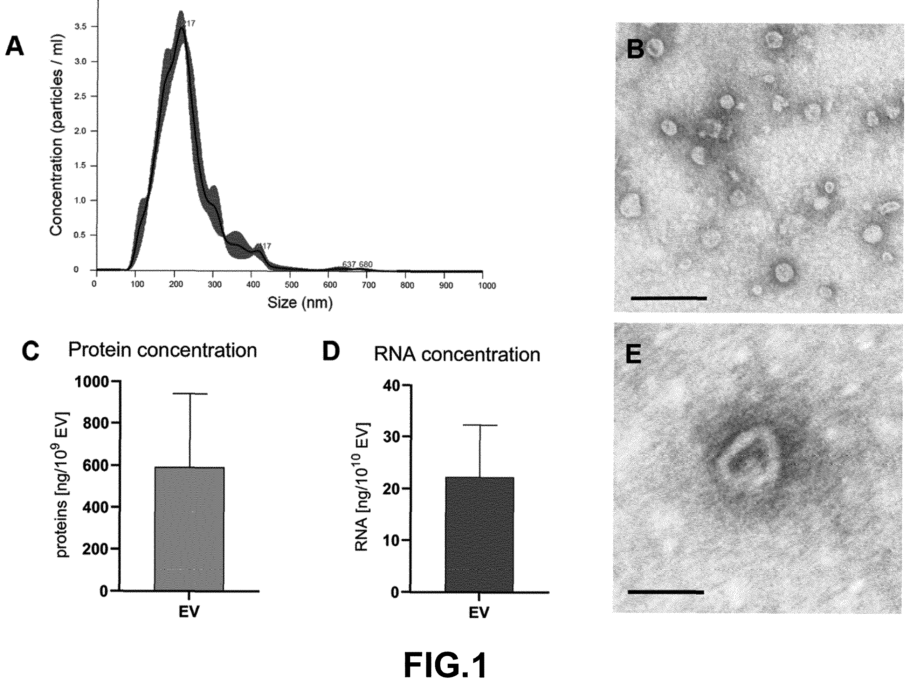

Figure 1 shows the characterization of orange-derived EVs in experimental example 1. (A) Representative image of NanoSight analysis and transmission electron microscopy photographs, (at original magnifications of x60,000, scale bar 500 nm (B) and x150,000, scale bar 200 nm(E)), showed a size in diameter typical of orange-derived EVs. The bar graphs show (C) the mean protein content of EVs expressed as nanograms (ng) of protein in 10⁹ EVs, and (D) the RNA content expressed as nanograms (ng) of RNA in 10¹⁰ EVs, as measured on several preparations of orange-derived EVs.

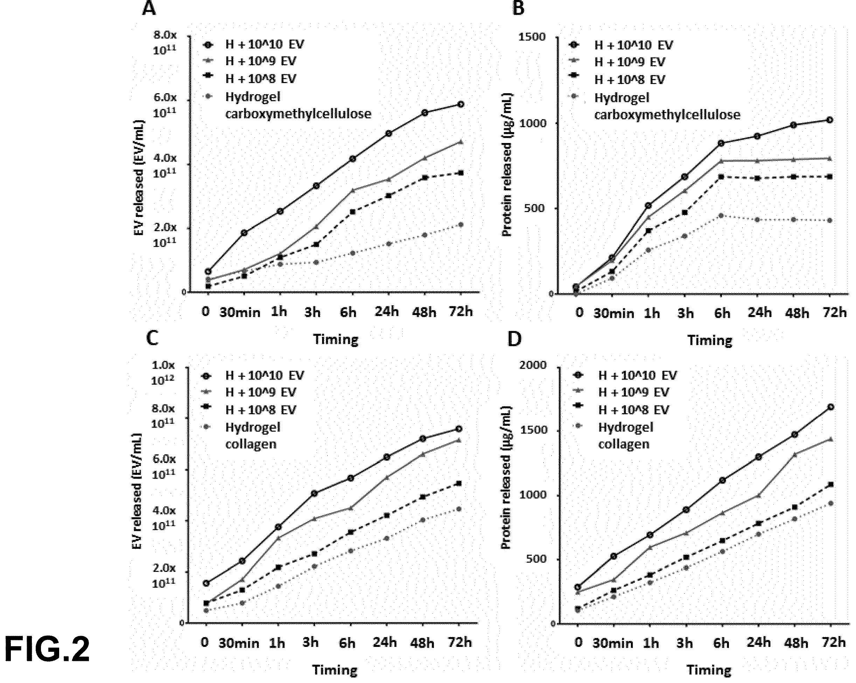

Figure 2 shows the release of orange-derived EVs from different hydrogels in experimental example 2. The release of EVs from hydrogels (carboxymethylcellulose and collagen) was evaluated as number of EVs (A for carboxymethylcellulose, C for collagen) and protein amount (B for carboxymethylcellulose, D for collagen) released over time. To perform the experiment, the hydrogel or the pharmaceutical composition, comprising hydrogel and EVs at different doses (10⁸, 10⁹ and 10¹⁰ EVs), were kept in a 24 well plate in direct contact with saline solution. The saline solution was analyzed for the presence of EVs at time 0 (0) and after 30 minutes (30 min), 1 hour (1h), 3 hours (3h), 6 hours (6h), 24 hours (24h), 48 hours (48h), 72 hours (72h). EVs and proteins were quantified in the saline solution at each timepoint by using the NanoSight instrument and the BCA kit, respectively. The amount of EVs released by hydrogel was expressed as number of EV/ml, whereas the amount of released protein was expressed as µg/ml. N=3 experiments were performed for each data set.

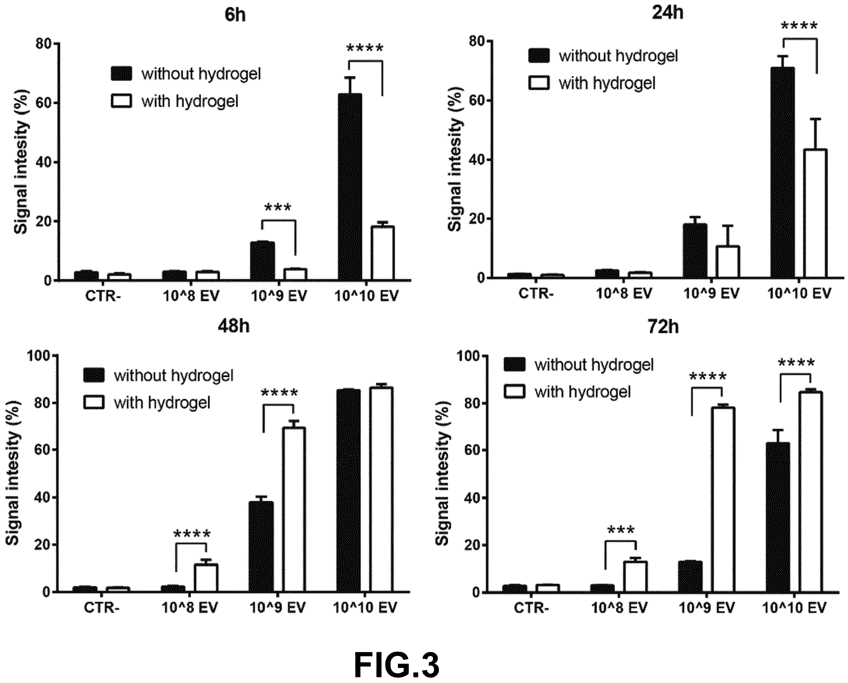

Figure 3 shows the incorporation of the orange-derived EVs of the pharmaceutical composition in target cells in experimental example 3. HMEC target cells were treated with mixtures of fluorescently labelled EVs (fluorochrome PKH26) and hydrogel (carboxymethylcellulose, collagen, ethylcellulose, chitosan, gelatin, PEG). EVs uptake in target cells was measured by Citofluorimetry at different time-points: 6 hours (6h), 24 hours (24h), 48 hours (48h) and 72 hours (72h). The EVs uptake is shown as percentage of signal intensity compared to target cells alone (negative control, CTR-). N=3 experiments were performed for each data set. CTR-: unstimulated endothelial cells. 10⁸, 10⁹ or 10¹⁰ EV: dose of orange-derived EVs used with or without hydrogel. The statistical significance was calculated comparing vesicle uptake of EVs alone or in combination with hydrogel. p: *** < 0.005; **** <0.001.

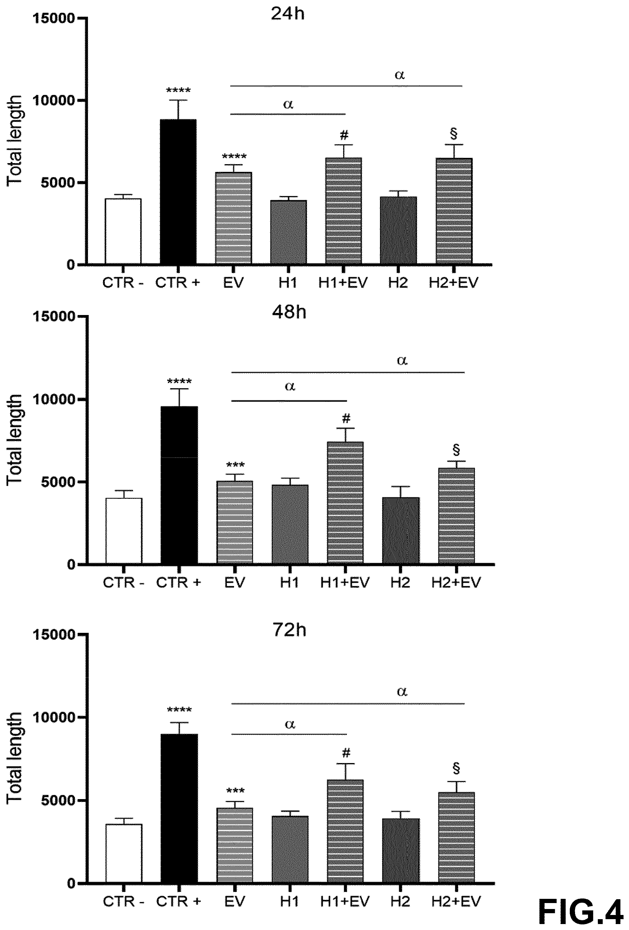

Figure 4 shows the ability of the pharmaceutical composition of the invention to promote angiogenesis on endothelial cells *in vitro* in experimental example 4. Tube formation assay was performed by stimulating endothelial cells with orange-derived EVs alone or hydrogel alone or the combination of EVs and hydrogel, for 24, 48 and 72 hours. The histograms show the total length of tube structure formed on Matrigel (mean ± SEM) upon stimulation carried out as above described. N=3 experiments were performed for each data set. CTR-: unstimulated endothelial cells. CTR+: medium supplemented with Fetal Bovine Serum and Growth Factors. EV: 10⁹ orange-derived EVs. H1: carboxymethylcellulose hydrogel alone. H1+EV: carboxymethylcellulose hydrogel with 10⁹ orange-derived EVs. H2: collagen hydrogel alone. H2+EV: collagen hydrogel with 10⁹ orange-derived EVs. The statistical significance was calculated comparing each condition with every other condition. p: **** <0.0001 vs. CTR-; # <0.0001 vs. H1; § <0.0001 vs. H2; α < 0.05 vs EV.

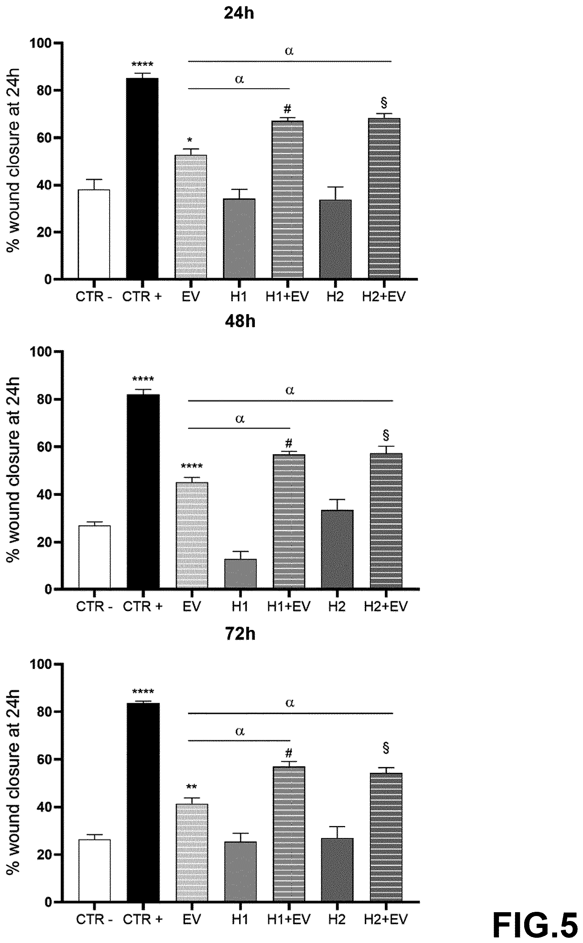

Figure 5 shows the ability of the pharmaceutical composition of the invention to promote endothelial cell migration *in vitro* in experimental example 4. Scratch test assay was performed by stimulating endothelial cells with orange-derived EVs alone or with hydrogel alone or the combination of EVs and hydrogel, for 24, 48 and 72 hours. The histograms show the percentage of wound closure (mean ± SEM) upon stimulation carried out as above described. N=3 experiments were performed for each data set. CTR-: unstimulated endothelial cells. CTR+: medium supplemented with Fetal Bovine Serum and Growth Factors. EV: 10⁹ orange-derived EVs. H1: carboxymethylcellulose hydrogel alone. H1+EV: carboxymethylcellulose hydrogel with 10⁹ orange-derived EVs. H2: collagen hydrogel alone. H2+EV: collagen hydrogel with 10⁹ orange-derived EVs. The statistical significance was calculated comparing each condition with every other condition. p: **** <0.0001 vs. CTR-; ** <0.01 vs. CTR-; * <0.05 vs. CTR-; # <0.0001 vs. H1; § <0.0001 vs. H2; α < 0.05 vs EV.

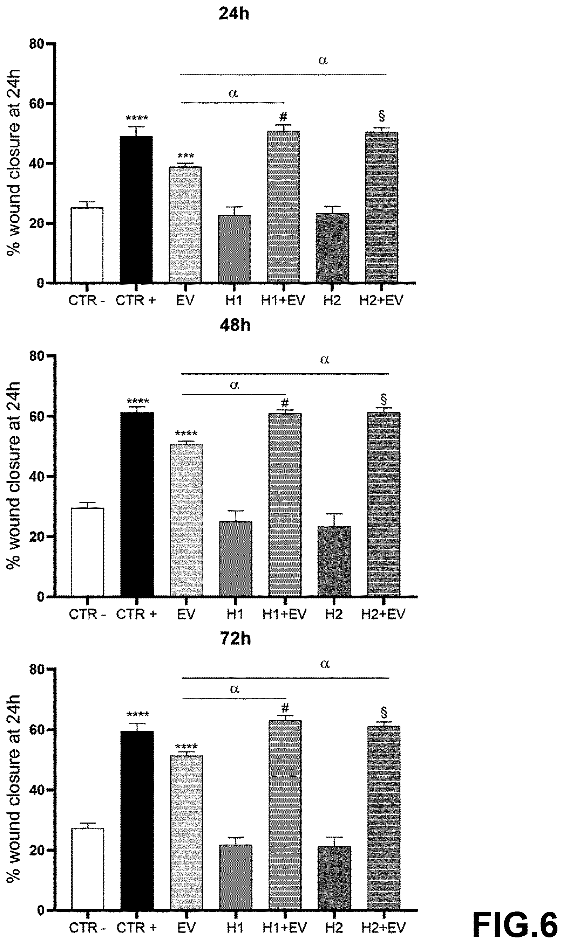

Figure 6 shows the ability of the pharmaceutical composition of the invention to promote fibroblast cell migration *in vitro* in experimental example 4. Scratch test assay was performed by stimulating fibroblast cells with orange-derived EVs alone or with hydrogel alone or the combination of EVs and hydrogel, for 24, 48 and 72 hours. The histograms show the percentage of wound closure (mean ± SEM) upon stimulation carried out as above described. N=4 experiments were performed for each data set. CTR-: unstimulated fibroblasts. CTR+: medium supplemented with 10% Fetal Bovine Serum. EV: 10⁹ orange-derived EVs. H1: carboxymethylcellulose hydrogel alone. H1+EV: carboxymethylcellulose hydrogel with 10⁹ orange-derived EVs. H2: collagen hydrogel alone. H2+EV: collagen hydrogel with 10⁹ orange-derived EVs. The statistical significance was calculated comparing each condition with every other condition. p: **** <0.0001 vs. CTR-; *** <0.001 vs. CTR-; # <0.0001 vs. H1; § <0.0001 vs. H2; α < 0.05 vs EV.

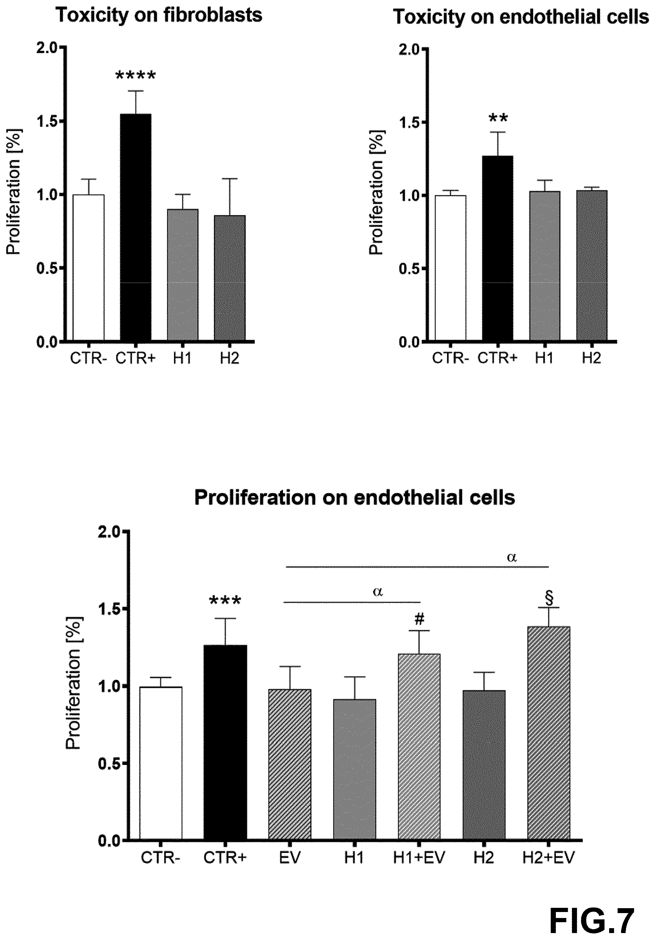

Figure 7 shows the absence of toxicity of different types of hydrogel on endothelial cells and fibroblasts *in vitro* in experimental example 4. Endothelial cells and fibroblasts were stimulated with carboxymethylcellulose hydrogel (H1) or collagen hydrogel (H2) and toxicity was measured as reduction of the proliferation rate assessed by BrdU proliferation assay, in comparison to the negative control. The graphs show the percentage of proliferation (mean ± SEM) for fibroblasts (upper-left) and endothelial cells (upper-right). N=3 experiments were performed for each data set. CTR-: unstimulated cells. CTR+: medium supplemented with 10% of Fetal Bovine Serum for fibroblasts, or medium supplemented with Fetal Bovine Serum and Growth Factors (Endogro) for endothelial cells. H1: carboxymethylcellulose hydrogel alone. H2: collagen hydrogel alone. The statistical significance was calculated comparing each condition with CTR-. p: **** <0.0001; ** <0.01.
Moreover, Figure 7 shows the ability of to promote endothelial cell proliferation *in vitro* in experimental example 4. Endothelial cells were stimulated with orange-derived EVs alone or hydrogel alone, or the combination of EVs and hydrogel, for 24, 48 and 72 hours. The graph in the lower part of the figure shows endothelial cells proliferation expressed as percentage (mean ± SEM) upon stimulation carried out for 24 hours as above described. N=3 experiments were performed for each data set. CTR-: unstimulated cells. CTR+: medium supplemented with 10% of Fetal Bovine Serum for fibroblasts, or medium supplemented with Fetal Bovine Serum and Growth Factors (Endogro) for endothelial cells. EV: 10⁹ orange-derived EVs. H1: carboxymethylcellulose hydrogel alone. H1+EV: carboxymethylcellulose hydrogel with 10⁹ orange-derived EVs. H2: collagen hydrogel alone. H2+EV: collagen hydrogel with 10⁹ orange-derived EVs. The statistical significance was calculated comparing each condition with every other condition. p: *** <0.001 vs. CTR-; # <0.05 vs. H1; § <0.001 vs. H2; α < 0.05 vs EV.

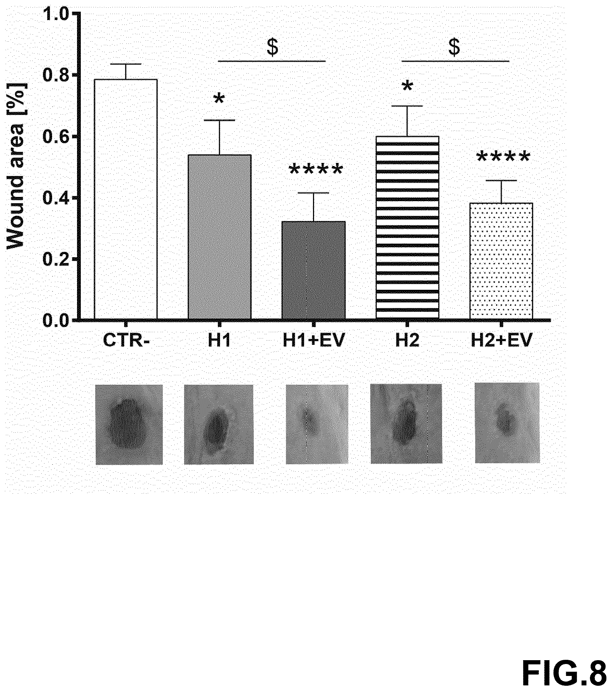

Figure 8 shows that the pharmaceutical composition of the invention accelerates tissue regeneration in an *in vivo* model of ulcers in experimental example 5. Ulcers were induced in mice and treated with carboxymethylcellulose (H1), carboxymethylcellulose with 10⁹ EVs (H1+EV), chitosan (H2), chitosan with 10⁹ EVs (H2+EV). As controls, untreated ulcers were used (CTR-). Wound area, i.e. the area of the wound still open, measured after 6 days of treatment is reported as percentage, compared to the original wound area at time 0. Representative images of treated wounds are shown below the graph. N=5 animals were used for each data set. CTR-: untreated wounds. The statistical significance was calculated comparing the effect of the different treatments with the untreated group (CTR-): p: * < 0.05; **** <0.001. The statistical significance was calculated by comparing the effect of each hydrogel with the same with EVs (H1 versus H1+EV, or H2 versus H2+EV): p: $<0.05.

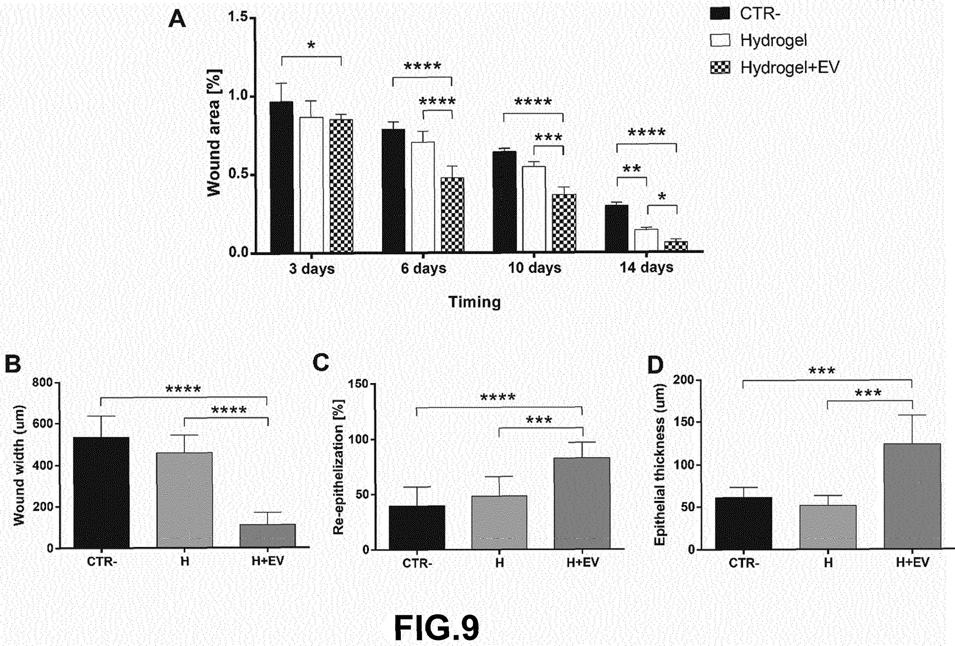

Figure 9 shows that the pharmaceutical composition of the invention accelerates tissue regeneration in a diabetic *in vivo* model of ulcers in experimental example 5. Ulcers were induced in mice and treated with carboxymethylcellulose (Hydrogel), carboxymethylcellulose with 10⁹ EVs (Hydrogel+EV). As controls, untreated ulcers were used (CTR-). (A) Wound area, i.e. the area of the wound still open, measured after 3, 6, 10 and 14 days of treatment is reported as percentage, compared to the original wound area at time 0. N=5 animals were used for each data set. CTR-: untreated wounds. The statistical significance was calculated comparing the effect of different treatments: p: * < 0.05; ** < 0.01; ***< 0.005; **** <0.001. (B, C, D) Results of histological analysis of wound tissues after 14 days of treatment. Hematoxylin and eosin staining was used to measure (B) the wound width intended as wound size (µm), (C) the percentage of re-epithelization as the percentage of newly formed epithelium in the wound in comparison to the original wound size, (D) the epithelial thickness of the wound (µm). The treatment with the combination of hydrogel and EVs was compared with the hydrogel alone (hydrogel) and untreated wounds (CTR-). N=5 animals were used for each data set. CTR-: untreated wounds. The statistical significance was calculated comparing the effect of different treatments: p: ***< 0.005; **** <0.001.

### Materials and methods

### Extracellular vesicles isolation

Extracellular vesicles were isolated from orange juice. Fruits of Citrus sinensis plants were squeezed and the juice was sequentially filtered using decreasing order of pores to remove fibers. EVs were then purified with differential ultracentrifugation or tangential flow filtration. For differential ultracentrifugation, the juice was centrifuged at 1,500 g for 30 minutes to remove debris and other contaminants. Then, EVs were purified by a first ultracentrifugation at 10,000 g followed by ultracentrifugation at 100,000 g for 1 hour at 4°C (Beckman Coulter Optima L-90K, Fullerton, CA, USA). The final pellet was resuspended with phosphate buffered saline added with 1% DMSO and filtered with 0.22 micrometer filters to sterilize.

Extracellular vesicles were used or stored at -80°C for long time. For tangential flow filtration, at first the juice was clarified by filtration with depth filter sheet discs Supracap 50 (Pall) to exclude fibers and debris. Then, the filtered juice was purified by concentration and diafiltration using a tangential flow filtration cassette TFF Omega (Pall Cadence). Finally, the retentate from tangential flow filtration was sterilized by filtration with a 0.2 nm filter.

### Nanoparticle tracking analysis (NTA)

Nanoparticle tracking analysis (NTA) was used to define the EV dimension in diameter and profile using the NanoSight LM10 system (NanoSight Ltd., Amesbury, UK), equipped with a 405 nm laser and with the NTA 3.1 analytic software. The Brownian movements of EVs present in the sample subjected to a laser light source were recorded by a camera and converted into size and concentration parameters by NTA through the Stokes-Einstein equation. Camera levels were for all the acquisition at 16 and for each sample, five videos of 30 s duration were recorded. Briefly, purified EVs were diluted 1:2000 in 1 ml vesicle-free saline solution (Fresenius Kabi, Runcorn, UK). NTA post-acquisition settings were optimized and maintained constant among all samples, and each video was then analyzed to measure EV mean, mode and concentration.

### Transmission electron microscopy (TEM)

Transmission electron microscopy of EVs was performed by loading EVs onto 200 mesh nickel formvar carbon coated grids (Electron Microscopy Science, Hatfield, PA) for 20 minutes. EVs were then fixed with a solution containing 2.5% glutaraldehyde and 2% sucrose and after repeated washings in distilled water, samples were negatively stained with NanoVan (Nanoprobes, Yaphank, NK, USA) and examined by Jeol JEM 1010 electron microscope.

### Protein extraction and quantification

Proteins were extracted from EVs by RIPA buffer (150 nM NaCl, 20 nM Tris-HCl, 0.1% sodium dodecyl sulfate, 1% deoxycholate, 1% Triton X-100, pH 7.8) supplemented with a cocktail of protease and phosphatase inhibitors (Sigma-Aldrich, St. Louis, Missouri, USA). The protein content was quantified by BCA Protein Assay Kit (Thermo Fisher Scientific, Waltham, Massachusetts, USA) following manufacturer's protocol. Briefly, 10 µl of sample were dispensed into wells of a 96-well plate and total protein concentrations were determined using a linear standard curve established with bovine serum albumin (BSA).

### RNA extraction and quantification

Total RNA was isolated from EVs and cells using the miRNeasy Mini Kit (Qiagen, Hilden, Germany) according to the manufacturer's protocol. RNA concentration of samples was quantified using spectrophotometer (mySPEC, VWR, Radnor, PA, USA).

### Hydrogel preparation

Hydrogels were produced by resuspending the hydrogel in water or saline solution. Hydrogels used included carboxymethylcellulose, collagen, ethylcellulose, chitosan, gelatin and PEG (Sigma-Aldrich, St. Louis, Missouri, USA). After extensive mixing the hydrogel was sterilized by filtering with filter 0.22 µm and ready to use. In order to combine hydrogel with orange-derived EVs, hydrogel was mixed with different doses of EVs (10⁸, 10⁹, 10¹⁰ EVs).

### Cell culture

Human microvascular endothelial cells (HMEC) were obtained by immortalization with simian virus 40 of primary human dermal microvascular endothelial cells. HMEC were cultured in Endothelial Basal Medium supplemented with bullet kit (EBM, Lonza, Basel, Switzerland) and 1 ml Mycozap CL (Lonza). Normal Human Dermal Fibroblasts (NHDF) are primary adult fibroblasts (Lonza). NHDF were cultured in FGM-2 Growth Media (Lonza) supplemented with bullet kit and 1 ml Mycozap PR (Lonza).

### Extracellular vesicles uptake

In order to evaluate the uptake into cells of orange-derived EVs, EVs were labeled with PKH-26 dye (Sigma-Aldrich, St. Louis, Missouri, USA) for 30 min at 37°C and washed by ultracentrifugation at 100,000 g for 2 h at 4°C using a 10 mL polycarbonate tube (SW 90 Ti rotor, Beckman Coulter Optima L-90 K ultracentrifuge). For uptake experiments, 25,000 HMEC cells/well were plated in 24-well plates and cultured with a transwell with pores of 0.4 µm containing saline solution (CTR-), the hydrogel or the combination of hydrogel and EVs. After each time point (6, 24, 48 and 72 hours) the transwell was removed, cells were extensively washed, detached with trypsin and their fluorescence was measured by FACS using the CytoFLEX flow cytometer with CytExpert software (Beckman Coulter Optima L-90K, Fullerton, CA, USA).

### In vitro tube formation assay

*In vitro* formation of tubes or capillary-like structures was studied on growth factor-reduced Matrigel (BD Bioscience, Franklin Lakes, NJ, USA) in 24-well plates. HMEC (25,000 cells/well) were seeded onto Matrigel-coated wells in DMEM alone (untreated control) or EndoGRO MV-VEGF medium (positive control), or in DMEM in the presence of hydrogel alone or hydrogel containing EVs (dose 10⁹ EVs). Each condition was performed in triplicate. After incubation for 24 hours, five random-field phase-contrast images (magnification, ×10) were recorded for each well. The total length of the network of tube structures was measured using ImageJ software. Results are expressed as the mean of the total length per each condition. The test was repeated with conditioned medium collected at 24, 48 and 72 hours.

### In vitro scratch-test assay

Endothelial cells (HMEC) and fibroblasts (NHDF) were seeded at a density of ~50x10³ cells/well in 24-well plates in the appropriate culture medium. When the cells reached complete confluence, scratch were created with a sterile tip to mimic a wound. Prior to stimulation (t=0), micrographs of the well were obtained using a Leica camera (Leica, Wetzlar, Germany). The cells were then stimulated with DMEM alone (negative control), or Endogro (Lonza) for HMEC or DMEM with 10% FBS for NHDF as positive control or DMEM containing EVs (dose 10⁹ EVs) or DMEM in the presence of hydrogel alone or hydrogel containing EVs (dose 10⁹ EVs). The 'wound closure' phenomenon was monitored for 24 hours, taking pictures using the Leica camera. Images were analyzed by ImageJ software (Bethesda, MD, USA) measuring the wound area. Results are expressed as a percentage of wound closure considering the wound area at t0 as 100% and calculating the corresponding area occupied by cells at 24 hours. The test was repeated with conditioned medium collected at 24, 48 and 72 hours.

### In vitro proliferation assay

Endothelial cells (HMEC) and fibroblasts (NHDF) were seeded in a 96 well plate at a density of 2,000 cells/well and left to adhere. The culture medium was replaced with DMEM to leave overnight. Then, cells were stimulated with DMEM alone (negative control), or Endogro (Lonza) for HMEC or DMEM with 10% FBS for NHDF as positive control or DMEM in the presence of hydrogel alone or hydrogel containing EVs (dose 10⁹ EVs) for 72 hours (. Each condition was performed in quadruplicate. Then 10 µl of BrdU labeling solution (BrdU colorimetric assay, Roche) were added to each well and the plate was incubated overnight. The effects of the stimuli were analyzed after 24 hours of incubation. The development of the assay was performed according with manufacturer's instruction. Absorbance was measured by an ELISA reader at 370 nm. The mean absorbance for each condition was calculated. Absorbance is directly proportional to proliferation rate. All mean absorbances were normalized for the mean of untreated control (CTR-), used as reference samples. The results show the relative proliferation rate compared to negative control, which is equal to 1.

### In vivo experiments

Normal male (BALB) or diabetic NOD.CB17-Prkdcscid/NCrCrl (NGS) male mice at 9 weeks old were anesthetized and back cutaneous hair was removed by electrical shaving. Diabetes was induced on NOD.CB17-Prkdcscid/NCrCrl (NGS) mice by intraperitoneal injection of streptozotocin (40 mg/kg) for 4 days and animals were treated after about 20 days of diabetes, measured as glycemia > 200. Four 6-mm diameter full-thickness skin wounds were created on each side of the midline using a disposable Biopsy Punch (PMD Medical). Each animal received different treatments on each wound. The day of the wounding was counted as day 0. Animals were monitored at different times (day 0, 3, 6 normal mice; and 0, 3, 6, 10, and 14 for diabetic mice) by wound measurement. At each time, the wounds received a fresh application of the treatments. At the last timepoint, mice were sacrificed and back skin wounds were collected for histological analysis. Histological analysis was performed using hematoxylin and eosin staining and was used to measure the wound width, the percentage of re-epithelization as the percentage of newly formed epithelium in the wound, the epithelial thickness of the wounds, using Imagej software.

### Statistical analysis

Data analysis was carried out with the software Graph Pad 8, demo version. Results are expressed as mean ± standard error (SEM). One-way analysis of variance (ANOVA) was used to substantiate statistical differences between groups, while Student's t-test was used for comparison between two samples. We used p < 0.05 as a minimal level of significance.

### Results/Examples

### Example 1

For their experiments, the inventors used orange-derived EVs. EVs were isolated by differential ultracentrifugation or tangential flow filtration. Results from the characterization of EVs obtained with the two methods were identical. Orange-derived EVs displayed a size in diameter in the range of 25-400 nm by Nanosight analysis, with a mean size in diameter of approximately 200 nm (Figure 1A). Orange-derived EVs showed a round morphology delimited by an electrondense membrane as demonstrated by transmission electron microscopy (TEM) analysis (Figure 1B and 1E). TEM analysis confirmed the mean size of EVs in diameter is approximately 200 nm.

In order to examine the content of orange-derived EVs, the protein and RNA content of EVs was measured. The protein concentration is shown in Figure 1C and demonstrate a heterogeneous protein content with a mean of 600 ng of proteins for 10⁹ EVs. The RNA concentration is shown in Figure 1D and demonstrate a variable RNA content with a mean of 22 ng of RNA for 10¹⁰ EVs.

Further analysis demonstrated that orange-derived EVs contain proteins characteristic of vesicles, such as HSP70, HSP80, glyceraldehyde-3-phosphate dehydrogenase (G3PD) and fructose-bisphosphate aldolase 6 (FBA6); and plant proteins, such as Patellin-3-like and clathrin heavy chain.

In addition, the lipid content of orange-derived EVs revealed a cargo of lipids, including 24-Propylidene cholesterol, Beta sitosterol, Glycidol stearate, Dipalmitin, Campesterol, Eicosanol, Eicosane, Hexadecane, Hexadecanol, Octadecane, Octadecanol, Tetradecane, Tetradecene, Valencene and Stearate.

### Example 2

The present inventors carried out experiments in order to assess that hydrogel preserves and releases orange-derived EVs in a controlled manner. For this purpose, the amount of EVs and the EV protein released in the microenvironment around the pharmaceutical composition was measured at increasing times (Figure 2). Briefly, the pharmaceutical composition of the invention was put in contact with saline solution and the release of EVs was measured in the saline solution at increasing time points. This analysis clearly demonstrated that hydrogels composed of either carboxymethylcellulose or collagen allow the release of EVs as particles already after 30 minutes (Figure 2 A, C). Moreover, the quantification of proteins confirmed that EVs are released from hydrogels already after 30 minutes (Figure 2 B, D). EVs release was already detectable after 30 minutes, increased over time, from 24 to 48 hours, and was still present after 72 hours. Similar data were obtained also with other hydrogels (ethylcellulose, chitosan, gelatin, PEG). Taken together, these results demonstrated that all hydrogels are able to preserve orange-derived EVs and allow their controlled release at increasing timings when in contact with cell or skin surface, starting already after 30 minutes of treatment and at least up to 3 days.

### Example 3

To perform their biological actions, orange-derived EVs have to be released by hydrogels and enter into target cells. In order to evaluate the functionality of EVs incorporated in the pharmaceutical composition, experiments were conducted to analyze the ability of these vesicles to enter (uptake) into target cells at different time points (6, 24, 48, and 72 hours) (Figure 3). For this type of experiment, increasing doses of orange-derived EVs (10⁸ EVs, 10⁹ EVs, 10¹⁰ EVs) labelled with a fluorochrome were included in the pharmaceutical composition.

The compositions were put into contact with target cells and EVs uptake in target cells (endothelial cells) was quantified by cytofluorimetric analysis. The uptake rate was evaluated by comparing the pharmaceutical composition of the invention with EVs alone (without hydrogel) and target cells alone (negative control, CTR-). The results shown in Figure 3 demonstrates that both the EVs included in the pharmaceutical composition and the EVs alone enter into target cells. Interestingly, the uptake of EVs alone was significantly higher compared to EVs included in the hydrogel after 6 and 24 hours of treatment. However, at increased time, i.e. at 48 and 72 hours, the trend was inverted and the combination of EVs with hydrogel allowed a significantly higher uptake in target cells. This effect increased with time, reaching the maximum peak at 72 hours. Taken together these data demonstrate that hydrogel can preserve EVs from degradation and allows a more efficient and prolonged release of these vesicles as well as their uptake into target cells, compared to EVs alone, already after 24 hours. Similar data were obtained with different hydrogels (carboxymethylcellulose, collagen, ethylcellulose, chitosan, gelatin, PEG).

### Example 4

In order to investigate the therapeutic potential of the pharmaceutical composition of the invention, *in vitro* functional tests were performed. In particular, the ability of the pharmaceutical composition including orange-derived EVs and hydrogel to promote endothelial cell angiogenesis and migration, as well as fibroblasts migration was tested *in vitro.*

The pharmaceutical composition containing hydrogel and orange-derived EVs was used to stimulate cells *in vitro* for 24, 48 and 72 hours. The results showed herein are obtained employing two kinds of hydrogels, carboxymethylcellulose hydrogel (H1) and collagen hydrogel (H2). Similar results were obtained with other types of hydrogels, including gelatin, chitosan, PEG and ethylcellulose.

EVs pro-angiogenic ability was tested by means of a tube formation assay, seeding endothelial cells on Matrigel (Figure 4) with the pharmaceutical composition containing hydrogel and orange-derived EVs or hydrogel alone or EVs alone, and incubating for 24, 48 and 72 hours. Endothelial cells were stimulated with orange-derived EVs alone (EV), hydrogel alone (H1, H2), or with the pharmaceutical composition comprising hydrogel and orange-derived EVs (H1+EV, H2+EV). All conditions were tested after EVs release for 24, 48, or 72 hours. A significant increase in total tube length was observed with EVs alone (EV) compared to untreated control (CTR-), and with the composition comprising hydrogel and EVs (H1+EV, H2+EV) compared to hydrogel alone (H1 and H2). Moreover, the composition comprising hydrogel and EVs (H1+EV, H2+EV) was shown to be significantly more effective in promoting angiogenesis than EVs alone (EV) and hydrogel alone (H1, H2). Such result was observed at each time point (24, 48 and 72 hours). This demonstrates that a composition comprising orange-derived EVs and hydrogel has a synergistic effect in promoting angiogenesis on endothelial cells and such pharmaceutical composition is significantly more effective than hydrogel alone or orange-derived EVs alone already after one day (24 hours) of treatment. Said effect is maintained over at least 3 days of treatment.

The effect of the pharmaceutical composition of the invention on endothelial cells and fibroblasts migration was assessed by performing a scratch on a monolayer of cells and measuring the migration of cells after 24 hours. The pharmaceutical composition containing hydrogel and orange-derived EVs was used to stimulate cells in vitro for 24, 48 and 72 hours.

The results shown are obtained using two kinds of hydrogels, i.e. carboxymethylcellulose hydrogel (H1) and collagen hydrogel (H2). Similar results were obtained with other types of hydrogels, including gelatin, chitosan, PEG and ethylcellulose.

The present inventors observed a significantly higher migration rate of endothelial cells (Figure 5) and fibroblasts (Figure 6) using the combination of orange-derived EVs and hydrogel (H1+EV and H2+EV) compared to EVs alone (EV), which are also effective in promoting cell migration but at a statistically significant lower extent, and hydrogel alone (H1 and H2), which is not effective and comparable to the untreated control. All conditions were tested after EVs release for 24, 48 or 72 hours and on both kind of cells. The experiments demonstrate that orange-derived EVs and hydrogel have a synergistic effect not only in the formation of new blood vessels but also in promoting migration of endothelial cells and such pharmaceutical composition is significantly more effective than hydrogel alone or orange-derived EVs alone already after one day (24 hours) of treatment. Said effect is maintained over at least 3 days of treatment (Figure 5). In fact, the migration of endothelial cells contributes to the formation of new blood vessels (angiogenesis) and this data support the pro-angiogenic activity of the pharmaceutical composition of the invention.

Similar results were obtained on fibroblast cells (Figure 6), demonstrating that the pharmaceutical composition comprising orange-derived EVs and hydrogel exploits a therapeutic effect also by promoting other cell types present in the lesion. Fibroblast, in fact, are involved in the regenerative process and the promotion of their migration contributes to accelerate the wound closure. The fact that the pharmaceutical composition of the invention can act on multiple cells in the lesion site supports its beneficial effect on accelerating tissue regeneration and wound closure.

In addition, the absence of toxicity of different types of hydrogel on endothelial cells and fibroblasts was assessed *in vitro* by a proliferation assay (Figure 7). The present inventors observed that carboxymethylcellulose hydrogel (H1) or collagen hydrogel (H2) do not affect and do not reduce the proliferation rate of fibroblasts (Figure, upper panel on the right) and endothelial cells (Figure 7, upper panel on the left). Similar results were also obtained with different types of hydrogel, including gelatin, chitosan, ethylcellulose and PEG. The toxicity of a compound is revealed by the reduction of proliferation of cells. The percentage of proliferation of fibroblasts and endothelial cells stimulated with H1 or H2 is comparable to the basal condition (unstimulated control, CTR-). This data demonstrate that hydrogels are not toxic and can be used in combination with orange-derived EVs for therapeutic purposes.

In addition, the present inventors observed that orange-derived EVs released from hydrogel (H1+EV and H2+EV) significantly increased proliferation of endothelial cells compared to orange-derived EVs alone (EV) or hydrogel alone (H1 and H2) (Figure 7, down panel). In the composition of the invention, orange-derived EVs and hydrogel have a synergistic effect in promoting proliferation of endothelial cells compared to untreated control (CTR-), and said composition is significantly more effective than hydrogel alone or orange-derived EVs alone. The results shown are obtained by employing two kinds of hydrogels, carboxymethylcellulose hydrogel (H1) and collagen hydrogel (H2). Similar results were obtained with other types of hydrogels, including gelatin, chitosan and ethylcellulose.

All experiments illustrated in example 4 confirm that EVs can be gradually released from hydrogel and maintain their biological effect at each time point (24, 48, and 72 hours). The pharmaceutical composition of the invention improves both hydrogel and EV biological activity already after few hours (6 hours) of treatment. Said effect is maintained over at least 3 days of treatment. In fact, the pharmaceutical composition containing orange-derived EVs and hydrogel is effective already after few hours of treatment and maintains the effectiveness for several days and guarantees a gradual distribution of EVs to recipient cells.

All results shown in experimental example 4 demonstrate that the therapeutic activity of the pharmaceutical composition comprising orange-derived EVs and hydrogel is mainly mediated by its pro-angiogenic activity. In fact, the combination of EVs and hydrogel promote the proliferation and migration of endothelial cells, and induce a higher formation of new blood vessels. Moreover, the combination of EVs and hydrogel acts also on other cell types involved in the lesion regeneration and present in the lesion, such as fibroblast cells. By promoting the migration of fibroblast cells, the combination of EVs and hydrogel allows fibroblast to migrate to the lesion site and deposit collagen, contributing to the promotion of wound closure.

### Example 5

To evaluate the therapeutic effect of the pharmaceutical composition, different hydrogels were combined with orange-derived EVs and their activity was tested on an *in vivo* model of ulcers (Figure 8). Ulcers were induced in a mice model and treated with carboxymethylcellulose (H1), carboxymethylcellulose and EVs (H1+EV), chitosan (H2), chitosan and EVs (H2+EV). After 3 days the medication was changed and the therapeutic effect was evaluated after 6 days of treatment. The effect was measured as percentage of wound area in comparison to the initial wound area at time 0 before treatment. Data clearly demonstrated that both hydrogels alone (carboxymethylcellulose and chitosan) were able to slightly reduce the wound area in comparison to untreated wound (CTR-) (Figure 8). Importantly, the combination of each hydrogel with orange-derived EVs significantly increased their therapeutic effect (H1+EV versus H1, and H2+EV versus H2). The results confirm the therapeutic efficacy of the pharmaceutical composition comprising hydrogel and orange-derived EVs. More specifically, this experiment demonstrates that the combination of hydrogel with EVs is more efficient in accelerating wound closure than hydrogel alone. For this reason, the pharmaceutical composition of the invention is particularly useful for the treatment of several kinds of lesions where the angiogenesis favors and accelerates the healing process, such as burns, fistulae, psoriasis, keratosis, keratitis, fissures, traumatic ulcers, mucosal lesions (such as traumatic lesions due to prothesis and such, mouth, decubital, genital mucosal lesions), dermatitis (including acne, eczema, seborrheic dermatitis, atopic dermatitis, contact dermatitis, dyshidrotic eczema, neurodermatitis, dermatitis herpetiformis), cellular damage induced by pro-apoptotic drugs aimed to treat pre-cancerous lesions (e.g. actinic keratosis).

With the aim to further analyze the therapeutic activity of the pharmaceutical composition of the invention, the regenerative effect of said composition was evaluated on *in vivo* mice model of diabetic ulcers (Figure 9). Diabetic ulcers, in fact, are more difficult to close than normal ulcers because of the diabetic condition that impairs tissue regeneration ability. The wound area was expressed as percentage of wound area in respect with the original area at time 0 before treatment (Figure 9A). The wounds were performed on diabetic mice and medications were changed every three days. The wound area measurement was performed at time 0 and after 3, 6, 10 and 14 days. The analysis clearly demonstrated that the treatment with the pharmaceutical composition of the invention was the most effective in accelerating wound closure in comparison to untreated wound (CTR-) and hydrogel alone (hydrogel) at each timepoint (Figure 9A).

Moreover, the treatment with the combination of hydrogel and EVs was the only able to induce a significant reduction of wound area already after 3 days of treatment (Figure 9A). The histological analysis of the tissues confirmed the higher therapeutic effect of the composition of the invention in comparison with hydrogel alone and untreated wounds (CTR-) (Figure 9 B, C, D). Tissue sections stained with hematoxylin and eosin were used to measure wound width (Figure 9B), the percentage of re-epithelization (Figure 9C) and the epithelial thickness (Figure 9D).

The wound width was significantly reduced upon treatment with the combination of hydrogel and EVs compared with the treatment with hydrogel alone or untreated control (Figure 9B).

The analysis of the percentage of re-epithelization demonstrated that the treatment with the combination of hydrogel and EVs induced a significantly higher formation of new epithelium in the wound compared with the treatment with hydrogel alone or untreated control (Figure 9C). The new epithelium is essential to the wound closure.

Finally, the tissue section subjected to the treatment with the combination of hydrogel and EVs presented a higher epithelial thickness in comparison to the treatment with hydrogel alone or untreated control (Figure 9D), showing that the regeneration is more efficient and allows the restoration of a normal higher epithelial thickness.

Taken together, all the data confirm the higher therapeutic effect of the pharmaceutical composition of the invention in accelerating wound closure of diabetic ulcers in comparison to the treatment with hydrogel alone, already after 3 days of treatment. This demonstrates that such pharmaceutical composition is particularly useful in the treatment of several kinds of lesions where the natural angiogenetic processes are impaired or compromised, as in case of diabetic foot ulcers, diabetic mucosal ulcers, dysmetabolic ulcers, or in presence of ischemic damages, including ischemic ulcers, such as pressure ulcers, arterial ulcers, venous ulcers, ischemic ulcers, exudative ulcers, traumatic ulcers, and other mucosal lesions.

## Claims

1. A pharmaceutical composition in the form of a hydrogel, comprising:
(i) orange-derived extracellular vesicles (EVs);
(ii) a polymer gelling agent;
(iii) water in an amount of at least 10% by weight of the total weight of the composition; and
(iv) optionally, pharmaceutically acceptable vehicles, excipients, and/or diluents,
wherein the orange-derived EVs are derived from one or more plants of the species *Citrus sinensis* and/or one or more fruits of said one or more *Citrus sinensis* plants,
wherein the orange-derived extracellular vesicles (EVs) are enclosed by a lipid bilayer membrane and are **characterized in that** they have a diameter ranging from 10 to 500 nm as measured by light scattering-based nanoparticle tracking analysis (NTA), and **in that** they show pro-angiogenic activity,
wherein the polymer gelling agent and the water form a hydrogel matrix in which the orange-derived EVs are dispersed, said orange-derived EVs being releasable from the hydrogel matrix,
for use in a tissue repair and/or regenerative therapeutic method.

2. The pharmaceutical composition for use according to claim 1, wherein the amount of the orange-derived EVs is in the range of from 10¹⁰ to 10¹² EVs/ml on the total volume of the composition.

3. The pharmaceutical composition for use according to claim 1 or 2, wherein the amount of the orange-derived EVs proteins is in the range of from 5 µg to 500 µg/ml on the total volume of the composition.

4. The pharmaceutical composition for use according to any one of claims 1 to 3, wherein the protein content of the orange-derived EVs is in the range of from 1 to 1 x10³ ng/10⁹ EVs.

5. The pharmaceutical composition for use according to any one of claims 1 to 4, wherein the RNA content of the orange-derived EVs is in the range of from 10 to 60 ng/10¹⁰ EVs.

6. The pharmaceutical composition for use according to any one of claims 1 to 5, wherein the polymer gelling agent is selected from the group consisting of cellulose and cellulose-based polymers, including carboxymethylcellulose, sodium carboxymethylcellulose, ethylcellulose, hydroxyethylcellulose, cellulose gum, collagen, hydrolyzed collagen, chitosan, gelatin, hyaluronic acid (HA), glycerol, polyethylene glycol (PEG), polysulfated glycosaminoglycan (PSGAG), glycerine, propylene glycol, calcium alginate, and any combination thereof.

7. The pharmaceutical composition for use according to any one of claims 1 to 6, further comprising a therapeutic agent selected from the group consisting of antibiotics, antibacterial agents, antifungal agents, anti-inflammatory agents, growth factors, pro-regenerative agents, and any combination thereof.

8. The pharmaceutical composition for use according to any one of claims 1 to 7, which has a pH comprised between 3.5 and 7.0.

9. The pharmaceutical composition for use according to any one of claims 1 to 8, wherein the regenerative therapeutic method is for the closure of a tissue lesion.

10. The pharmaceutical composition for use according to any one of claims 1 to 9, wherein the therapeutic treatment of a disease or condition is selected from the group consisting of ischemic ulcers, such as pressure ulcers, arterial ulcers, venous ulcers, diabetic ulcers, ischemic ulcers, exudative ulcers, dysmetabolic ulcers, traumatic ulcers, mucosal lesions such as diabetic lesions, burns, fistulae, psoriasis, keratosis, keratitis, fissures, traumatic ulcers, mucosal lesions including traumatic lesions due to prothesis and mouth, decubital, genital mucosal lesions, dermatitis including acne, eczema, seborrheic dermatitis, atopic dermatitis, contact dermatitis, dyshidrotic eczema, neurodermatitis, dermatitis herpetiformis, and cellular damage induced by pro-apoptotic drugs aimed to treat pre-cancerous lesions including actinic keratosis.

11. The pharmaceutical composition for use according to any of claims 1 to 10, which is suitable for topical administration or for administration by injection.

12. The pharmaceutical composition for use according to claim 11, wherein the treatment comprises the release of the orange-derived EVs from the hydrogel matrix over a period of at least 1 day.

13. The pharmaceutical composition for use according to claim 12, wherein the release of the orange-derived EVs from the hydrogel matrix is performed over a period of at least 7 days.

14. A pharmaceutical composition for use according to any one of claims 1 to 11, which is prepared by a method comprising the steps of:
(i) dissolving a polymer gelling agent in a predetermined amount of water to obtain a hydrogel matrix, wherein the pre-determined amount of water represents at least 10% by weight of the total weight of the pharmaceutical composition;
(ii) adding orange-derived extracellular vesicles (EVs) as defined in any one of claims 1, 2, 3, 4, 5 or 6 to the hydrogel matrix obtained in step (i); and
(iii) mixing said orange-derived EVs with the hydrogel matrix, thereby obtaining the pharmaceutical composition in the form of a hydrogel in which the EVs are dispersed in the hydrogel matrix.

## Patentansprüche

1. Pharmazeutische Zusammensetzung in Form eines Hydrogels, umfassend:
(i) aus Orangen gewonnene extrazelluläre Vesikel (EVs);
(ii) ein polymeres Geliermittel;
(iii) Wasser in einer Menge von mindestens 10 Gewichtsprozent des Gesamtgewichts der Zusammensetzung; und
(iv) gegebenenfalls pharmazeutisch annehmbare Träger, Hilfsstoffe und/oder Verdünnungsmittel, wobei die von Orangen gewonnenen EVs von einer oder mehreren Pflanzen der Spezies *Citrus sinensis* und/oder einer oder mehreren Früchten der genannten *Citrus* sinensis-Pflanze(n) gewonnen werden,
wobei die aus Orangen gewonnenen extrazellulären Vesikel (EVs) von einer Lipid-Doppelschichtmembran umschlossen sind und **dadurch gekennzeichnet sind, dass** sie einen Durchmesser im Bereich von 10 bis 500 nm aufweisen, wie durch eine auf Lichtstreuung basierende Nanopartikel-Verfolgungsanalyse (NTA) gemessen, und dass sie eine proangiogene Aktivität aufweisen,
wobei das polymere Geliermittel und das Wasser eine Hydrogelmatrix bilden, in welcher die von Orangen gewonnenen EVs dispergiert sind, wobei die genannten von Orangen gewonnenen EVs aus der Hydrogelmatrix freisetzbar sind,
zur Verwendung in einem Gewebereparatur- und/oder regenerativen therapeutischen Verfahren.

2. Pharmazeutische Zusammensetzung zur Verwendung gemäß Anspruch 1, wobei die Menge der aus Orangen gewonnenen EVs im Bereich von 10¹⁰ bis 10¹² EVs/ml, bezogen auf das Gesamtvolumen der Zusammensetzung, beträgt.

3. Pharmazeutische Zusammensetzung zur Verwendung nach Anspruch 1 oder 2, wobei die Menge der aus Orangen gewonnenen EVs-Proteine im Bereich von 5 µg bis 500 µg/ml, bezogen auf das Gesamtvolumen der Zusammensetzung, beträgt.

4. Pharmazeutische Zusammensetzung zur Verwendung nach einem der Ansprüche 1 bis 3, wobei der Proteingehalt der aus Orangen gewonnenen EVs im Bereich von 1 bis 1 x10³ ng/10⁹ EVs beträgt.

5. Pharmazeutische Zusammensetzung zur Verwendung nach einem der Ansprüche 1 bis 4, wobei der RNA-Gehalt der aus Orangen gewonnenen EVs im Bereich von 10 bis 60 ng/10¹⁰ EVs beträgt.

6. Pharmazeutische Zusammensetzung zur Verwendung nach einem der Ansprüche 1 bis 5, wobei das polymere Geliermittel ausgewählt ist aus der Gruppe bestehend aus Cellulose und cellulosebasierten Polymeren, einschließlich Carboxymethylcellulose, Natriumcarboxymethylcellulose, Ethylcellulose, Hydroxyethylcellulose, Cellulosegummi, Collagen, hydrolysiertem Collagen, Chitosan, Gelatine, Hyaluronsäure (HA), Glycerin, Polyethylenglycol (PEG), polysulfatiertem Glycosaminoglycan (PSGAG), Glycerin, Propylenglycol, Calciumalginat und jeder Kombination davon.

7. Pharmazeutische Zusammensetzung zur Verwendung nach einem der Ansprüche 1 bis 6, ferner umfassenddie ferner ein therapeutisches Mittel, ausgewählt aus der Gruppe bestehend aus Antibiotika, antibakteriellen Mitteln, antimykotischen Mitteln, entzündungshemmenden Mitteln, Wachstumsfaktoren, pro-regenerativen Mitteln und einer beliebigen Kombination davon.

8. Die pharmazeutische Zusammensetzung zur Verwendung nach einem der Ansprüche 1 bis 7, welche einen pH-Wert zwischen 3,5 und 7,0 aufweist.

9. Pharmazeutische Zusammensetzung zur Verwendung nach einem der Ansprüche 1 bis 8, wobei das regenerative therapeutische Verfahren dem Verschluss einer Gewebeläsion dient.

10. Pharmazeutische Zusammensetzung zur Verwendung nach einem der Ansprüche 1 bis 9, wobei die therapeutische Behandlung einer Krankheit oder eines Zustands ausgewählt ist aus der Gruppe bestehend aus ischämischen Geschwüren, wie Druckgeschwüren, arteriellen Geschwüren, venösen Geschwüren, diabetischen Geschwüren, ischämischen Geschwüren, exsudativen Geschwüren, dysmetabolischen Geschwüren, traumatischen Geschwüren, Schleimhautläsionen, wie diabetischen Läsionen, Bums, Fisteln, Psoriasis, Keratose, Keratitis, Fissuren, traumatische Geschwüre, Schleimhautläsionen einschließlich traumatischer Läsionen aufgrund von Prothesen und Mund, Dekubitus, genitale Schleimhautläsionen, Dermatitis einschließlich Akne, Ekzem, seborrhoische Dermatitis, atopische Dermatitis, Kontaktdermatitis, dyshidrotisches Ekzem, Neurodermitis, Dermatitis herpetiformis und durch pro-apoptotische Medikamente induzierte Zellschäden zur Behandlung von präkanzerösen Läsionen einschließlich aktinischer Keratose.

11. Pharmazeutische Zusammensetzung zur Verwendung nach einem der Ansprüche 1 bis 10, die zur topischen Verabreichung oder zur Verabreichung durch Injektion geeignet ist.

12. Pharmazeutische Zusammensetzung zur Verwendung nach Anspruch 11, wobei die Behandlung die Freisetzung der aus Orange gewonnenen EVs aus der Hydrogelmatrix über einen Zeitraum von mindestens 1 Tag umfasst.

13. Pharmazeutische Zusammensetzung zur Verwendung nach Anspruch 12, wobei die Freisetzung der aus Orangen gewonnenen EVs aus der Hydrogelmatrix über einen Zeitraum von mindestens 7 Tagen durchgeführt wird.

14. Pharmazeutische Zusammensetzung zur Verwendung nach einem der Ansprüche 1 bis 11, welche durch ein die folgenden Schritte umfassendes Verfahren hergestellt wird:
(i) Auflösen eines polymeren Geliermittels in einer vorbestimmten Menge Wasser, um eine Hydrogelmatrix zu erhalten, wobei die vorbestimmte Menge Wasser mindestens 10 Gew.-% des Gesamtgewichts der pharmazeutischen Zusammensetzung repräsentiert;
(ii) Zugeben von aus Orangen gewonnenen extrazellulären Vesikeln (EVs), wie in einem der Ansprüche 1, 2, 3, 4, 5 oder 6 definiert, zu der in Schritt (i) erhaltenen Hydrogelmatrix; und
(iii) Mischen der genannten aus Orangen gewonnenen EVs mit der Hydrogelmatrix, wodurch die pharmazeutische Zusammensetzung in Form eines Hydrogels erhalten wird, in welchem die EVs in der Hydrogelmatrix dispergiert sind.

## Revendications

1. - Composition pharmaceutique sous la forme d'un hydrogel, comprenant :
(i) des vésicules extracellulaires (VE) dérivées de l'orange ;
(ii) un agent gélifiant polymère ;
(iii) de l'eau dans une quantité d'au moins 10 % en poids du poids total de la composition ; et
(iv) facultativement, des véhicules, excipients et/ou diluants pharmaceutiquement acceptables,
dans laquelle les VE dérivées de l'orange sont dérivées d'une ou de plusieurs plantes de l'espèce *Citrus sinensis* et/ou d'un ou de plusieurs fruits de ladite ou desdites plantes *Citrus sinensis*,
dans laquelle les vésicules extracellulaires (VE) dérivées de l'orange sont enfermées par une membrane bicouche lipidique et sont **caractérisées en ce qu'**elles ont un diamètre se situant dans la plage de 10 à 500 nm, tel que mesuré par analyse de suivi de nanoparticules (NTA) basée sur la diffusion de la lumière, et **en ce qu'**elles présentent une activité pro-angiogénique,
dans laquelle l'agent gélifiant polymère et l'eau forment une matrice d'hydrogel dans laquelle les VE dérivées de l'orange sont dispersées, lesdites VE dérivées de l'orange pouvant être libérées de la matrice d'hydrogel,
pour une utilisation dans une méthode thérapeutique de réparation et/ou de régénération des tissus.

2. - Composition pharmaceutique pour une utilisation selon la revendication 1, dans laquelle la quantité d'VE dérivées de l'orange se situe dans la plage de 10¹⁰ à 10¹² VE/ml sur le volume total de la composition.

3. - Composition pharmaceutique pour une utilisation selon l'une des revendications 1 ou 2, dans laquelle la quantité des protéines des VE dérivées de l'orange se situe dans la plage de 5 µg à 500 µg/ml sur le volume total de la composition.

4. - Composition pharmaceutique pour une utilisation selon l'une quelconque des revendications 1 à 3, dans laquelle la teneur en protéines des VE dérivées de l'orange se situe dans la plage de 1 à 1 x 10³ ng/10⁹ VE.

5. - Composition pharmaceutique pour une utilisation selon l'une quelconque des revendications 1 à 4, dans laquelle la teneur en ARN des VE dérivées de l'orange se situe dans la plage de 10 à 60 ng/10¹⁰ VE.

6. - Composition pharmaceutique pour une utilisation selon l'une quelconque des revendications 1 à 5, dans laquelle l'agent gélifiant polymère est choisi dans le groupe constitué par la cellulose et les polymères à base de cellulose, comprenant la carboxyméthylcellulose, la carboxyméthylcellulose sodique, l'éthylcellulose, l'hydroxyéthylcellulose, la gomme de cellulose, le collagène, le collagène hydrolysé, le chitosane, la gélatine, l'acide hyaluronique (HA), le glycérol, le polyéthylène glycol (PEG), le glycosaminoglycane polysulfaté (PSGAG), la glycérine, le propylène glycol, l'alginate de calcium et toute combinaison de ceux-ci.

7. - Composition pharmaceutique pour une utilisation selon l'une quelconque des revendications 1 à 6, comprenant en outre un agent thérapeutique choisi dans le groupe constitué par les antibiotiques, les agents antibactériens, les agents antifongiques, les agents anti-inflammatoires, les facteurs de croissance, les agents pro-régénératifs et toute combinaison de ceux-ci.

8. - Composition pharmaceutique pour une utilisation selon l'une quelconque des revendications 1 à 7, dont le pH est compris entre 3,5 et 7,0.

9. - Composition pharmaceutique pour une utilisation selon l'une quelconque des revendications 1 à 8, dans laquelle la méthode thérapeutique régénérative est destinée à la fermeture d'une lésion tissulaire.

10. - Composition pharmaceutique pour une utilisation selon l'une quelconque des revendications 1 à 9, dans laquelle le traitement thérapeutique d'une maladie ou d'une affection est choisi dans le groupe constitué par les ulcères ischémiques, tels que les escarres, les ulcères artériels, les ulcères veineux, les ulcères diabétiques, les ulcères ischémiques, les ulcères exsudatifs, les ulcères dysmétaboliques, les ulcères traumatiques, les lésions des muqueuses, telles que les lésions diabétiques, les brûlures, les fistules, le psoriasis, la kératose, la kératite, les fissures, les ulcères traumatiques, lésions des muqueuses comprenant les lésions traumatiques dues aux prothèses et les lésions buccales, de décubitus et des muqueuses génitales, la dermatite comprenant l'acné, l'eczéma, la dermatite séborrhéique, la dermatite atopique, la dermatite de contact, l'eczéma dyshidrotique, la neurodermatite, la dermatite herpétiforme et les dommages cellulaires induits par les médicaments pro-apoptotiques destinés à traiter les lésions précancéreuses comprenant la kératose actinique.

11. - Composition pharmaceutique pour une utilisation selon l'une quelconque des revendications 1 à 10, qui convient pour une administration topique ou pour une administration par injection.

12. - Composition pharmaceutique pour une utilisation selon la revendication 11, dans laquelle le traitement comprend la libération des VE dérivées de l'orange à partir de la matrice d'hydrogel sur une période d'au moins 1 jour.

13. - Composition pharmaceutique pour une utilisation selon la revendication 12, dans laquelle la libération des VE dérivées de l'orange à partir de la matrice d'hydrogel est effectuée sur une période d'au moins 7 jours.

14. - Composition pharmaceutique pour une utilisation selon l'une quelconque des revendications 1 à 11, qui est préparée par un procédé comprenant les étapes consistant à :
(i) dissoudre un agent gélifiant polymère dans une quantité prédéterminée d'eau pour obtenir une matrice d'hydrogel, la quantité prédéterminée d'eau représentant au moins 10 % en poids du poids total de la composition pharmaceutique ;
(ii) ajouter des vésicules extracellulaires (VE) dérivées de l'orange telles que définies à l'une quelconque des revendications 1, 2, 3, 4, 5 ou 6 à la matrice d'hydrogel obtenue à l'étape (i) ; et
(iii) mélanger lesdites VE dérivées de l'orange avec la matrice d'hydrogel, permettant ainsi d'obtenir la composition pharmaceutique sous la forme d'un hydrogel dans lequel les VE sont dispersées dans la matrice d'hydrogel.
